# EUROPÄISCHE PATENTSCHRIFT

(11) **EP 1 069 892 B1**
(45) Veröffentlichungstag und Bekanntmachung des Hinweises auf die Patenterteilung: **07.08.2002**
(21) Anmeldenummer: 99914523.8
(22) Anmeldetag: 18.03.1999
(51) Int. Cl.: A61K 9/70, A61K 47/02, A61K 31/40, A61K 31/27

(54) **FEUCHTIGKEITSAKTIVIERBARES THERAPEUTISCHES SYSTEM**
THERAPEUTIC SYSTEM WHICH CAN BE MOISTURE-ACTIVATED
SYSTEME THERAPEUTIQUE POUVANT ETRE ACTIVE PAR L'HUMIDITE

(30) Priorität: 30.03.1998 DE 19814087
(43) Veröffentlichungstag der Anmeldung: 24.01.2001
(73) Patentinhaber: LTS Lohmann Therapie-Systeme AG, 56626 Andernach (DE)
(72) Erfinder: BRACHT, Stefan, D-56299 Ochtendung (DE)
(74) Vertreter: Flaccus, Rolf-Dieter, Dr.
(86) Internationale Anmeldenummer: EP9901802
(87) Internationale Veröffentlichungsnummer: WO9949853

(56) Entgegenhaltungen:
- WO-A-94/07468
- WO-A-97/11696
- AT-B- 392 587
- CHEMICAL ABSTRACTS, vol. 117, no. 16, 19. Oktober 1992 (1992-10-19) Columbus, Ohio, US; abstract no. 157659, YANAGIBASHI, NORIO ET AL: "Transdermal patches for perianal diseases" XP002110472 & JP 04 124128 A (LION CORP., JAPAN)

## Beschreibung

Die Erfindung betrifft ein therapeutisches System zur zeitlichen und mengenmäßig steuerbaren Abgabe von wenigstens einem therapeutischen Wirkstoff an einen menschlichen oder tierischen Organismus mittels Diffusion durch die Haut oder Schleimhaut, wobei der Wirkstoff für die Herstellung und

Lagerung zunächst in einer ersten Zustandsform vorliegt, in der er chemisch stabil und für die Haut oder Schleimhaut nicht ausreichend permeabel ist, während er am Applikationsort durch Zutritt von Feuchtigkeit in eine zweite Zustandsform umgewandelt wird, in der er für die Diffusion durch die Haut oder Schleimhaut geeignet ist und in den Organismus aufgenommen wird.

Anlaß zum Einsatz eines aktivierbaren Systems besteht immer dann, wenn diejenige chemische Zustandsform eines Wirkstoffs, die sich am besten für die Abgabe an den Körper eignet, nicht identisch ist mit derjenigen Form, die für die Produktion und Lagerung eines therapeutischen Systems optimal geeignet ist.

Therapeutische Systeme, die durch Feuchtigkeit aktiviert werden, sind bekannt und beispielsweise in US 4,781,924; EP 0 316 065 B1; DE 38 81 340 beschrieben.

Nur ein Teil dieser Dokumente betrifft feuchtigkeitsaktivierbare Systeme, bei denen die Aktivierung eine chemische Umwandlung des Wirkstoffes beinhaltet.

Diese umfassen beispielsweise Systeme, bei denen Wirkstoff zunächst in einer Zustandsform enthalten ist, die durch die Oberfläche des Systems nicht in therapeutisch ausreichenden Mengen pro zeit diffundieren kann.

Neben dem Wirkstoff ist daher ein "aktivierendes Mittel" erforderlich, das im ersten Zustand wasserfrei vorliegt, während es unter Aufnahme von Hautfeuchtigkeit in einen zweiten, hydratisierten und gelösten Zustand übergeht.

Im gelösten Zustand ist dann das aktivierende Mittel in der Lage, Wirkstoff in eine zweite Form zu überführen, welche durch die Oberfläche des Systems in therapeutisch ausreichenden Mengen pro Zeit zu diffundieren vermag.

Darüber hinaus ist in den genannten Dokumenten ein Aufbau von Systemen beschrieben, bei denen der Aktivator von vornherein in gelöster Form vorliegt, jedoch in Mikrokapseln eingeschlossen ist, welche im Ausgangszustand den Aktivator nicht an die Umgebung abgeben. Die Aktivierung umfaßt eine Zerstörung der Mikrokapseln, vorzugsweise durch Zerbrechen oder Schmelzen. Eine Aktivierung durch Hautfeuchtigkeit wird bei dieser Modifizierung nicht beschrieben. Die bekannten Systeme sind im Aufbau kompliziert und verursachen deshalb vergleichsweise hohe Fertigungskosten bei zum Teil unbefriedigendem Aktivierungsprozeß.

Der Erfindung liegt ausgehend vom vorgenannten Stand der Technik die Aufgabe zugrunde, feuchtigkeitsaktivierbare Systeme zu vereinfachen und dadurch die Herstellungskosten zu reduzieren.

Ein weiterer Teil der Aufgabe ist es, den Aktivierungsprozeß zu beschleunigen sowie
eine Erhöhung der Abgabeleistung an Wirkstoff aus solchen Systemen zu erreichen.

Diese Aufgaben werden mit der Erfindung überraschend mit den Merkmalen von Anspruch 1 gelöst. Dadurch, daß der Aktivator weder in wasserfreiem Zustand, noch in gelöster Form im System vorliegt, und daß er statt dessen in einer hydratisierten, aber ungelösten chemischen Zustandsform eingesetzt wird, wobei es sich vorzugsweise um Hydrate des Aktivators handelt, weisen diese einen kristallinen Ordnungszustand auf, wobei sie in diesem Zustand zusätzlich zum Aktivator Wasser in einem definierten Mengenverhältnis mitführen.

Diese hydratisierte Form des Aktivators liegt als ungelöster Feststoff im System vor.

Diese ungelöste Form ist praktisch nicht in der Lage, den Wirkstoff von einer ersten chemischen Zustandsform in eine zweite zu überführen. Sie geht erst bei Zutritt von Hautfeuchtigkeit in Lösung und wird dann in der gewünschten Weise gegenüber dem Wirkstoff aktiv. Durch das von Anfang an im Kristall des Aktivators mitgeführte Wasser wird der Prozeß der Aktivierung beschleunigt und im Gesamtausmaß deutlich verbessert.

Pevorzugten Einsatz findet das erfindungsgemäße System bei der Herstellung von transdermalen therapeutischen Systemen (TTS).

Da der Aktivator im ungelösten Zustand inaktiv ist, gelingt es sogar, Wirkstoff und Aktivator in ein und dieselbe Schicht eines TTS einzuarbeiten, die gleichzeitig auch selbst haftklebende Eigenschaften haben kann.

Insbesondere durch diesen letztgenannten "Drug-in-Adhesive"-Aufbau gelingt es, den Wirkstoff erstaunlich schnell und in großer Menge freizusetzen, selbst wenn die wasserfreien Formen des Aktivators eingesetzt werden.

Damit werden vereinfachte TTS-Aufbauten möglich bei gleichzeitig beschleunigter und erhöhter Wirkstofffreisetzung.

Die veränderlichen chemischen Zustandsformen betreffen speziell das Säure-Base-Gleichgewicht, in dem der betreffende Wirkstoff vorliegt.

Unter den verwendeten Wirkstoffen befindet sich eine große Zahl von Verbindungen, die saure oder basisch reagierende Molekülgruppen beinhalten; die überwiegende Mehrzahl besitzt basische Eigenschaften.

Bei den basischen Gruppen handelt es sich in aller Regel um primäre, sekundäre oder tertiäre Amine. Diese funktionellen Gruppen sind reaktiv und können an einer Vielzahl von Reaktionen teilnehmen (z.B. Oxidationsprozesse), die leicht zu einer Zersetzung des Wirkstoffs führen.

Werden diese Gruppen durch Reaktion mit einer Säure in ein Salz überführt, so führt dies sehr häufig zu einer deutlichen Verbesserung der chemischen Stabilität.

Weiterhin weisen die Salze in aller Regel höhere Schmelzpunkte auf als die freien Basen. Eine bei Raumtemperatur flüssig vorliegende Base kann durch Umwandlung in ein Salz sogar in den Festzustand übergehen.

Neben der Erhöhung des Schmelzpunktes findet durch Salzbildung immer eine Erniedrigung der Flüchtigkeit statt. Schließlich nimmt durch die Salzbildung die Wasserlöslichkeit praktisch immer deutlich zu, während die Löslichkeit in organischen Lösungsmitteln parallel dazu deutlich abnimmt.

Die vorgenannten Eigenschaften der Salze basischer Wirkstoffe führen dazu, daß in der pharmazeutischen Forschung und Entwicklung in einer großen Mehrzahl der Fälle dem Salz eines basischen Wirkstoffes der Vorzug als Rohstoff gegenüber der freien Base gegeben wird.

Für die TTS-Entwicklung ergibt sich daraus häufig ein Nutzen durch die höhere Stabilität der Salze.

Eine besondere Rolle spielen aber auch der erhöhte Schmelzpunkt und die erniedrigte Flüchtigkeit der Salzform, insbesondere deshalb, weil viele Verfahren zur Herstellung von TTS immer einen Abschnitt bei deutlich erhöhter Arbeitstemperatur im Bereich typischerweise zwischen 60 und 120 °C umfassen.

Bei lösungsmittelhaltigen Beschichtungsverfahren betrifft dies die Trocknung zur Entfernung der prozeßbedingten Lösungsmittel. Beim lösungsmittelfreien Heißschmelzverfahren wird die Produktmasse zur Erniedrigung der Viskosität zeitweise stark aufgeheizt.

Bei der Herstellung von TTS ergeben sich folglich sehr leicht Probleme mit einem niedrigschmelzenden und/oder flüchtigen Wirkstoff in der Form der freien Base. Die Salzformen bieten in dieser Hinsicht erhebliche Vorteile.

Unter den vorgenannten Gesichtspunkten ist es bei der TTS-Entwicklung häufig wünschenswert, einen Wirkstoff in der Salzform zu verarbeiten.

Die Wirkstoffsalze eignen sich jedoch, verglichen mit den Nicht-Salz-Formen, wenig für die transkutane Verabreichung. Beispielsweise hat die Barriere der menschlichen Haut einen vorwiegend lipophilen Charakter. Gegenüber stark polaren, wasserlöslichen Verbindungen ist sie deshalb nahezu undurchlässig, weshalb die Überführung eines Wirkstoffes in ein Salz in nahezu allen Fällen mit einer Verschlechterung der Aufnahme über die Haut einhergeht.

Damit ergeben sich häufig widersprüchliche Anforderungen an den Wirkstoff, die nicht von einer seiner chemischen Zustandsformen allein erfüllt werden können.

Eine Lösung des Problems bieten aktivierbare Formen von TTS.

Unter der Aktivierung eines TTS ist hier zu verstehen, daß die hinsichtlich Verarbeitung und Lagerung günstige Salzform des enthaltenen Wirkstoffes eingesetzt und erst bei der späteren Anwendung des TTS unter bestimmten äußeren Einflüssen in die Nicht-Salz-Form überführt wird, die sich durch bessere Hautgängigkeit auszeichnet. Unter den möglichen äußeren Einflüssen wird bevorzugt die Aufnahme von Feuchtigkeit nach Applikation auf die Hautoberfläche betrachtet.

Die menschliche Haut gibt Feuchtigkeit auf zwei Wegen ab:

Über die als Epidermis bezeichnete äußerste Hautschicht erfolgt ständig ein passiver, durch Diffusion bedingter Austritt von Wasserdampf, der transepidermale Wasserverlust. Über die Schweißdrüsen wird dagegen aktiv Wasserdampf abgegeben, bei stärkerem Schwitzen tritt Wasser sogar in flüssiger Form auf der Hautoberfläche aus.

Ein auf die Haut appliziertes TTS ist dieser austretenden Hautfeuchtigkeit ausgesetzt und kann je nach seiner Beschaffenheit mehr oder weniger viel Feuchtigkeit aufnehmen. Eine wichtige Rolle spielt dabei die der Haut abgewandte äußere Rückschicht eines TTS. Je weniger wasserdampfdurchlässig diese Schicht ist, um so ausgeprägter ist der Feuchtigkeitsstau im TTS und es kommt schließlich zur Okklusion.

Dieser Feuchtigkeitsstau kann zur Aktivierung einer chemischen Umwandlungsreaktion genutzt werden.

Zwei wasserlösliche oder zumindest wasserquellbare Reaktionspartner A und B, die im TTS in trockener, praktisch ungelöster Form vorliegen, können erst nach Zutritt von Feuchtigkeit unter Auflösung oder zumindest Anlösung miteinander in Reaktion treten.

Dieses Prinzip ist durch die Brausetablette allgemein bekannt, die erst durch Zutritt von Wasser im Rahmen einer Säure-Base-Reaktion Kohlendioxid bildet.

Handelt es sich um eine Säure-Base-Reaktion, so ist Wasser lediglich als Lösungsmittel für die Reaktanden erforderlich. Es wird jedoch bei der Reaktion nicht vei aucht. Eine geringe Menge Wasser genügt daher bereits, um den Prozeß zu starten und kontinuierlich weiterlaufen zu lassen.

Um nun den salzförmig vorliegenden Wirkstoff im Rahmen einer Säure-Base-Reaktion aus dem Salz freizusetzen, bedarf es eines hier als Aktivator bezeichneten Reaktionspartners. Therapeutische Systeme, die sich dieses Aktivierungsprinzip zunutze machen, wurden bereits in den oben zitierten US 4,781,924, EP O 316 065 B1, DE 3881 340 beschrieben.

Dort wurde jedoch davon ausgegangen, daß das aktivierende Agenz in einem ausdrücklich wasserfreien Zustand vorliegen muß.

Das aktivierende Agens ist erst in einem zweiten Zustand hydratisiert bzw. geht in Lösung, nachdem es bei der Anwendung Feuchtigkeit aufgenommen hat, oder aber alternativ, nachdem es aus einem Vorrat innerhalb des Systems Feuchtigkeit aufgenommen hat.

Es wurde nun überraschend gefunden, daß es nicht erforderlich ist, den Aktivator in einer wasserfreien Form im System vorrätig zu halten. Auch wenn der Aktivator in einer hydratisierten, aber ungelösten Form vorliegt, unterbleibt offenbar die Reaktion mit dem Wirkstoffsalz während der Herstellung und Lagerung des Systems nahezu völlig.

Viele als Aktivatoren geeignete Hilfsstoffe liegen typischerweise gar nicht wasserfrei vor. Sie müssen erst getrocknet oder in der (häufig teureren) wasserfreien Form bezogen und entsprechend gelagert werden. Dies betrifft beispielsweise die in dem zitierten Stand der Technik genannten Stoffe Natriumcarbonat, Natriummonohydrogen- und

Natriumorthophosphat, die in der wasserfreien Form sogar hygroskopisch sind.

Die entsprechenden Nachteile entfallen bei Einsatz der hydratisierten Formen.

Ein im System bereits hydratisiert vorliegender Aktivator erbringt darüber hinaus sogar Vorteile hinsichtlich des Aktivierungsverlaufes bei Zutritt von Feuchtigkeit.

Es wurde gefunden, daß unter Verwendung bereits hydratisierter Aktivatoren die Geschwindigkeit und das Ausmaß des Aktivierungprozesses gegenüber den wasserfreien Aktivatoren verbessert ablaufen.

Dieser Vorgang ist nicht zu verwechseln mit einer in WO 94/07468 beschriebenen Vorgehensweise, nach der der Wirkstoff in seiner wasserlöslichen Salzform und ein anorganisches Silikat zunächst in Wasser miteinander vermischt, diese wäßrige Lösung in eine Polymerlösung eingearbeitet und daraus TTS hergestellt werden. Diese enthalten nach einem Trocknungsschritt erfindungsgemäß das Silikat in hydratisierter Form innig mit dem Wirkstoff gemischt als innere disperse Phase in einem umgebenden Polymer. Dabei ist der wasserlösliche Wirkstoff erfindungsgemäß sogar teilweise in der wäßrigen Phase des Silikates gelöst.

Dagegen beinhalten die Systeme nach der Erfindung nur Aufbauten, bei denen Wirkstoff und Aktivator getrennt voneinander in einer gemeinsamen oder auch in verschiedenen Matrices dispergiert vorliegen. Ein dispergierendes Agens ist nicht notwendigerweise enthalten.

Als Aktivatoren mit wasserhaltigem kristallinen Aufbau kommen anorganische oder organische Verbindungen in Betracht, die in wäßriger Lösung sauer oder basisch reagieren.

Für die Umwandlung der basisch reagierenden Zustandsform eines Wirkstoffs in die sauer reagierende Zustandsform wird ein sauer reagierender Aktivator eingesetzt. Basisch reagierende Aktivatoren dienen dagegen zur Umwandlung der sauer reagierenden Zustandsform des Wirkstoffs in die basisch reagierende Form.

Als basisch reagierende Aktivatoren kommen, ohne Anspruch auf Vollständigkeit, folgende Verbindungen in Betracht:

Basische Silikate, basische Phosphate, Citrate, Tartrate, Succinate, basische Salze der Ethylendiamintetraessigsäure, Carbonate, Hydrogencarbonate und Hydroxide.

Diese Verbindungen kommen als Alkali-, Erdalkali- oder Aluminiumsalze zum Einsatz.

Weiterhin möglich sind Verbindungen, die aus mehr als einem der vorgenannten Anionen und mehr als einem der vorgenannten Metallkationen gleichzeitig in einem kristallin definierten Mischzustand aufgebaut sind. Auch können die vorgenannten Anionen und Kationen im Kristallgitter mit noch weiteren, hier nicht genannten Ionen kombiniert sein.

Als sauer reagierende Aktivatoren kommen, ohne Anspruch auf Vollständigkeit, folgende Verbindungen in Betracht:

Dihydrogenphosphate, Citronensäure und Dihydrogencitrate, Weinsäure und Hydrogentartrate, Trihydrogensalze der Ethylendiamintetraessigsäure sowie Hydrogensulfate.

Diese Verbindungen kommen gegebenenfalls als Alkali-, Erdalkali- oder Aluminiumsalze zum Einsatz.

Alle solchen Aktivatoren werden vorzugsweise in einer Form verwendet, die im kristallinen Zustand definierte Mengenanteile Wasser mit sich führt.

Auch Mischungen verschiedener Aktivatoren sind möglich und können zur Einstellung eines bestimmten Aktivierungsverhaltens sinnvoll sein. Die Mischung kann auch verschieden stark wasserhaltige kristalline Zustandsformen ein und desselben Aktivators betreffen, um das Aktivierungsverhalten zu modulieren.

Eine Mischung verschiedener Aktivatoren kann auch sinnvoll sein, wenn der Wirkstoff gegenüber allzu starken Basen instabil sein sollte. Über die Mischung kann in solchen Fällen ein Optimum zwischen erwünschten, aktivierenden, und unerwünschten, zersetzenden Einflüssen eingestellt werden.

Bei der überwiegenden Mehrheit der heutigen pharmazeutischen Wirkstoffe handelt es sich um basische Substanzen, saure Wirkstoffe stellen dagegen die Minderheit dar.

Die basischen Wirkstoffe werden sehr häufig in Form ihrer chemisch stabileren und nichtflüchtigen, wasserlöslichen Salze verwendet. Dies sind z.B. die Hydrochloride und Sulfate.

Da die freien Basen in aller Regel besser hautgängig sind als die ionischen Salze, kommt der Umwandlung der Salze basischer Wirkstoffe in die freien Wirkstoffbasen besondere Bedeutung zu. Diese Umwandlung kann unter Einsatz eines basischen Aktivators in einem feuchtigkeitsaktivierbaren TTS erfolgen.

Basische Aktivatoren finden daher im Rahmen der vorliegenden Erfindung eine besondere Beachtung:

Unter den möglichen basischen Aktivatoren eignen sich speziell die Silikate und Phosphate.

Bevorzugten Einsatz finden unter den Silikaten das Natriummetasilikat-Pentahydrat sowie die hydratisierten Formen von Natriumtrisilikat.

Ebenfalls besonders geeignet sind die Hydrate des Magnesiumtrisilikates, typischerweise das Pentahydrat.

Unter den Phosphaten eignen sich die basischen Monohydrogenphosphate und Orthophosphate sowie Pyrophosphate.

Dies sind insbesondere Dinatriummonohydrogenphosphat-Dihydrat, -Heptahydrat und -Dodecahydrat sowie Trinatriumphosphat-Hexahydrat und -Dodecahydrat. Außerdem eignet sich Tetranatriumdiphosphat-Decahydrat.

Weiterhin kommen Trikaliumphosphat-Monohydrat und - Trihydrat sowie Magnesiumhydrogenphosphat-Trihydrat in Betracht.

Besonderes Interesse gilt weiterhin solchen Aktivatoren, die ein inneres Puffersystem aufweisen und somit nur kontrolliert basisch reagieren.

Dies betrifft speziell die auch als säurebindende Mittel vom Menschen innerlich einnehmbaren Verbindungen Magnesiumcarbonathydroxid und Aluminiummagnesiumhydroxidsulfat. Beide Verbindungen können in verschiedenen Zusammensetzungen auftreten, die wegen ihres Gehaltes an Wasser im Kristall gut zur Herstellung der erfindungsgemäßen TTS geeignet sind.

Stellvertretend für Aluminiummagnesiumhydroxidsulfat wird hier Magaldrat (INN) genannt. Nach den Angaben der USP 23 handelt es sich um ein Produkt wechselnder Zusammensetzung mit der allgemeinen Formel Al₅Mg₁₀(OH)₃₁(SO₄)₂ •X H₂O. Stellvertretend für Magnesiumcarbonathydroxid steht Hydrotalcit (INN). Nach den Angaben des Merck-Index (12. Auflage 1996) handelt es sich bei Magnesiumcarbonathydroxid im allgemeinen um (MgCO₃)₄ Mg(OH)₂ •5H₂O und bei Hydrotalcit im speziellen um Al₂O₃ 6MgO CO₂ •12H₂O.

Neben diesen Beispielen aus der anorganischen Chemie seien noch folgende Aktivatoren auf organischer Basis erwähnt: Trinatriumcitrat Dihydrat (C₆H₅Na₃O₇•2H₂O), Magnesiumcitrat Tetradecahydrat (C₁₂H₁₀Mg₃O₁₄•14 H₂O), Tetranatriumedetat Dihydrat (C₁₄H₁₂N₂Na₄O₈•2H₂O), Kaliumnatriumtartrat Tetrahydrat (C₄H₄KNaO₆•4H₂O) und Dinatriumsuccinat Hexahydrat (C₄H₄Na₂O₄•6H₂O).

Bei der Auswahl eines geeigneten Aktivators spielen allgemein die Entwässerungstemperaturen eine große Rolle. Die üblichen Herstellungsprozesse für ein TTS umfassen in aller Regel einen Trocknungsprozeß bei lösungsmittelhaltigen Beschichtungsverfahren oder aber einen Schmelzvorgang bei der Herstellung im Heißschmelzverfahren.

Die temperaturbedingte vollständige Abgabe von Wasser aus dem Aktivator sollte möglichst oberhalb dieser Verarbeitungstemperaturen liegen bzw. die Verarbeitungstemperaturen sollten unterhalb der vollständigen Entwässerungstemperatur des Aktivators gehalten werden.

Die Differenz sollte mindestens 1-5°C, besser 5-20°C und idealerweise mehr als 20°C betragen.

Unter diesem Gesichtspunkt eignen sich unter den vorgenannten Aktivatoren ganz besonders die Silikate und Phosphate.

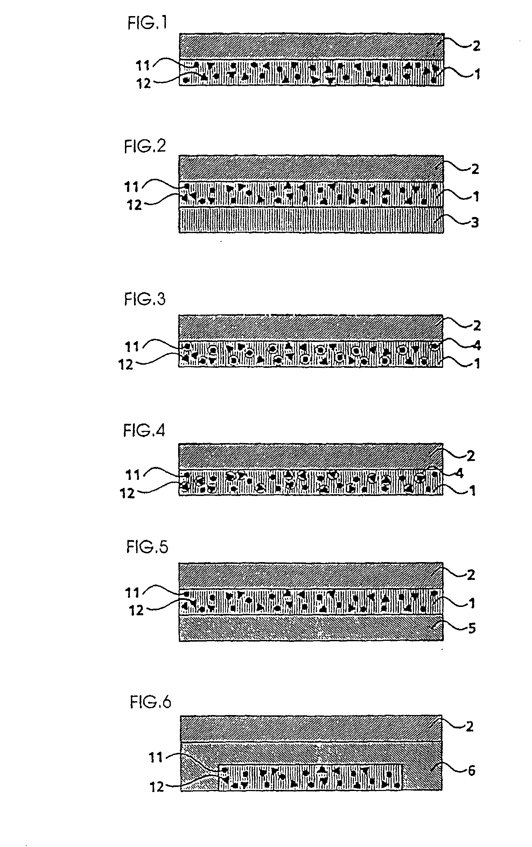

Der Aufbau von feuchtigkeitsaktivierbaren transdermalen therapeutischen Systemen unter Einsatz hydratisierter Aktivatoren läßt sich vielseitig gestalten. Dies wird durch die FIG. 1-13 illustriert, die nicht maßstabsgetreu sind, sondern beispielhafte Schichtaufbauten zeigen.

Im Rahmen des einfachsten und zugleich bevorzugten Aufbaues befinden sich der Wirkstoff in einer zur Herstellung und Lagerung des TTS geeigneten Salzform sowie der Aktivator im ungelösten Zustand in ein und derselben Schicht des TTS. Bei einer anderen Konstruktion werden Wirkstoff und Aktivator in getrennte Schichten des TTS eingearbeitet.

In allen Fällen können Steuerschichten implementiert werden. Diese Steuerschichten werden entweder zwischen den Wirkstoff und den Aktivator gebracht oder aber sie befinden sich zwischen dem Wirkstoff- sowie Aktivatorreservoir und der Hautoberfläche.

Im ersten Fall wird der Zutritt des Aktivators nach Feuchtigkeitsaufnahme und Lösung zum Wirkstoff gesteuert oder aber der Zutritt des Wirkstoffs nach Feuchtigkeitsaufnahme und Lösung zum Aktivator.

Im zweiten Fall wird nach Feuchtigkeitsaufnahme die Freisetzung des Wirkstoffs in seiner bereits aktivierten, hautgängigen Form an die Hautoberfläche gesteuert.

Alternativ kann es sich bei den Steuerschichten auch um eine Steuerung der Feuchtigkeitsaufnahme des Systems insgesamt oder einzelner seiner Schichten handeln.

Zur Steuerung eignen sich in diesem Sinne solche Schichten, deren Wasserdampfdurchlässigkeit unterhalb derjenigen Rate pro Zeit liegt, mit der Feuchtigkeit von der Haut typischerweise abgegeben wird.

Um eine Feuchtigkeitsaktivierung zu erzielen, müssen bei allen vorstehenden Aufbauten aus der Haut austretendes Wasser bzw. Wasserdampf im TTS gestaut werden. Die Rückschicht des TTS ist daher vorzugsweise wasserdampfundurchlässig ausgebildet.

Diese Eigenschaft besitzen insbesondere Filme aus Polyethylenterephthalat, Polyethylen, Polypropylen, Polyvinylchlorid (PVC) und Polyvinylidenchlorid (PVDC), aber auch solche aus Ethylenvinylacetat-Copolymer (EVA) mit vorzugsweise geringem Vinylacetat-Anteil <10 %. Auch Filme aus sehr elastischen Kohlenwasserstoffpolymeren wie Polyisobutylen, Polyisopren oder die Blockcopolymere von Styrol und Isopren bzw. Butadien kommen infrage.

Als Rückschicht können auch mehrschichtige Verbundmaterialien (Laminate) eingesetzt werden, bei denen nur eine Schicht aus den genannten Polymeren besteht.

Schließlich kann auch die Rückschicht als Steuermembran eingesetzt werden, indem eine definierte, geringe Wasserdampfdurchlässigkeit eingestellt wird. Dadurch ist eine definierte Verzögerung des Feuchtigkeitsstaues und damit der Aktivierung möglich.

Beispielhafte Schichtaufbauten werden nachfolgend unter Bezug auf die Figuren beschrieben.

Werden Wirkstoff (11) und Aktivator (12) in eine Schicht eingebracht, so besitzt diese Schicht (1) in einem besonders bevorzugten Aufbau gleichzeitig haftklebende Eigenschaften, so daß keine separate Kleberschicht benötigt wird (System A, FIG. 1).

Das System A besteht dann nur aus einer ablösbaren Schutzschicht, einer haftklebenden Schicht, die Wirkstoff und Aktivator enthält, sowie aus einer Rückschicht (2).

Nach Applikation auf die Haut kommt es unter Feuchtigkeitsaufnahme zu einer Auflösung des Aktivators, der bis dahin in einem hydratisierten, aber ungelösten Zustand vorlag. Der gelöste Aktivator diffundiert zum salzförmig vorliegenden Wirkstoff und wandelt diesen in die Form einer freien Säure oder freien Base um. Diese freie Form diffundiert durch die haftklebende Schicht zur Haut und wird dort in den Körper aufgenommen.

Alternativ kann durch die Feuchtigkeitsaufnahme aber auch das Wirkstoffsalz gelöst werden und zum Aktivator diffundieren. Dort wird es in die freie Säure oder Base des Wirkstoffs umgewandelt, die die besser hautgängige Form des Wirkstoffs darstellt und anschließend durch Diffusion an die Haut abgegeben wird.

Das System A kann alternativ mit einer Steuerschicht (3) ausgerüstet werden (System B, FIG.2).

Die Steuerschicht steuert entweder die Feuchtigkeitsaufnahme und damit die Aktvierung selbst oder aber die Wirkstoffabgabe und damit das Freisetzungsverhalten nach Aktivierung. In dem dargestellten Aufbau besitzt die Steuerschicht gleichzeitig haftklebende Eigenschaften und dient zur Fixierung des Systems auf der Haut.

Wahlweise kann diese Steuerfunktion auch von einer den Wirkstoff oder den Aktivator im dispergierten Zustand umhüllenden Schicht (4) übernommen werden (Systeme C und D, FIG.3 und 4).

Falls der Wirkstoff oder der Aktivator mit der haftklebenden Schicht inkompatibel sein sollten, ist die Hinzufügung einer separaten haftklebenden Schicht (5) auf der hautzugewandten Seite zu bevorzugen (System E, FIG. 5). Diese Hinzufügung einer Haftkleberschicht ist sinngemäß auch auf die Systeme A-D anwendbar.

Die zusätzliche haftklebende Schicht muß nicht vollflächig ausgebildet sein. Die Verankerung des Systems auf der Haut kann auch durch eine über die Reservoirschicht nach außen überstehende haftklebende Schicht (6) bewirkt werden (System F, FIG. 6). Auch dieses Vorgehen ist auf die Systeme A-D übertragbar.

Mit dem Ziel, ein bestimmtes Aktivierungs- oder Freisetzungsverhalten zu erzielen, kann es erforderlich sein, den Wirkstoff und den Aktivator in getrennte Schichten des TTS einzuarbeiten.

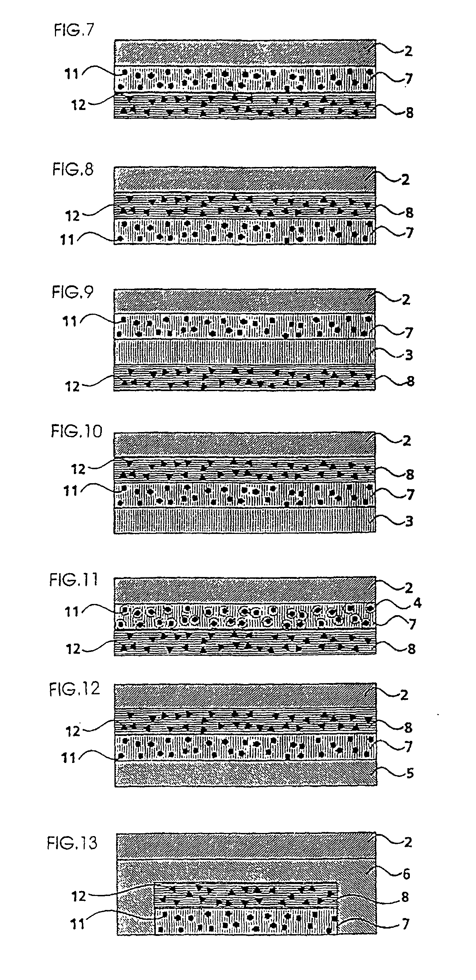

Im bevorzugten Fall ist eine dieser beiden Reservoirschichten, also die den Wirkstoff enthaltende Schicht (7) oder die den Aktivator enthaltende Schicht (8), gleichzeitig die haftklebende Schicht, die die Verankerung auf der Haut ermöglicht. Entweder befindet sich der Wirkstoff in einer hautnäheren Schicht als der Aktivator (System G, FIG. 7) oder umgekehrt (System H, FIG. 8).

Bei einem solchen Aufbau wird unter Feuchtigkeitsaufnahme der Aktivator gelöst, diffundiert in die Nachbarschicht zum Wirkstoff in der Salzform und setzt daraus die Base oder Säure frei. Diese freie Form diffundiert dann zur Haut und tritt über diese in den Körper ein. Alternativ kann es auch zur Auflösung des Wirkstoffs kommen, der in die Nachbarschicht zum Aktivator diffundiert und von diesem zur freien Base oder Säure umgewandelt wird.

Um den Zutritt von Wirkstoff zum Aktivator oder den umgekehrten Vorgang zu steuern, kann eine Steuerschicht (3) sinnvoll sein, die zwischen den beiden Reservoirschichten für Wirkstoff und Aktivator angebracht ist (System I, FIG. 9). Das Prinzip ist sinngemäß auch auf das System H anwendbar.

Die Steuerschicht kann auch als hautnächste Schicht (3) angebracht werden und bei gleichzeitig haftklebenden Eigenschaften der Verankerung des Gesamtsystems auf der Haut dienen (System J, FIG. 10).

Anstelle der Einführung separater Steuerschichten können in Analogie zu den Systemen C und D auch der Wirkstoff oder der Aktivator im dispersen Zustand mit einer steuernden Schicht (4) umhüllt werden (System K, FIG. 11).

Falls weder Wirkstoff noch Aktivator mit einem geeigneten Haftkleber kompatibel sein sollten, kann die Einführung einer separaten Haftkleberschicht (5) erforderlich sein (System L, FIG. 12).

Dieser Aufbau ist sinngemäß auf die Systeme G-K übertragbar.

Schließlich kann anstelle einer vollflächigen, hautseitigen Haftkleberschicht auch eine nur im Randbereich die Reservoirschichten überlappende Haftkleberschicht (6) in Analogie zu System F verwendet werden (System M, FIG. 13). Auch dieses Vorgehen ist sinngemäß auf die Systeme G-K übertragbar.

Vorzugsweise besitzt ein erfindungsgemäß konstruiertes TTS den im folgenden beschriebenen Aufbau:

Die Gesamtkonstruktion folgt Fig.1. Der Wirkstoff und das aktivierende Mittel in ihrer jeweils ersten chemischen Zustandsform liegen als Feststoffe dispergiert in einer einzigen Matrixschicht nebeneinander vor. diese Matrixschicht hat zugleich haftklebende Eigenschaften und dient zur Fixierung des Systems auf der Haut. Die haftklebende Matrix basiert vorzugsweise auf einem Silikonkautschuk.

Der Wirkstoff ist in seiner nichtionischen Form eine chemische Base, und diese basische Form weist eine geringe chemische Stabilität auf.

Das aktivierende Mittel ist in seiner ersten Zustandsform ein basisch reagierender Stoff, vorzugsweise handelt es sich um ein Erdalkalimetasilikat oder Erdalkalitrisilikat in einer hydratisierten Form.

Die Rückschicht des TTS besteht aus einer nahezu wasserdampfundurchlässigen Folie, vorzugsweise aus Polyethylenterephthalat (PET) oder einem Laminat aus 2 Schichten von denen eine aus PET besteht.

Die Primärverpackung des TTS besitzt eine mgölichst hohe Sperrwirkung gegenüber wasserdampt, uum eine vorzeitige Aktivierung während der Lagerung zu unterbinden.

### Beispiele

### 1. Aktivatoren

Unter den beschriebenen basischen Aktivatoren eignen sich wegen ihres ausgeprägten Wasserbindungsvermögens besonders die Silikate und Phosphate.

Deren Entwässerungsverhalten wurde an 3 Beispielsubstanzen durch Difference Scanning Calorimetry (DSC) untersucht. Diese thermische Analyse gibt Auskunft über die unter Energieaufnahme stattfindenden Stufen der Entwässerung und die jeweils typische Temperatur.

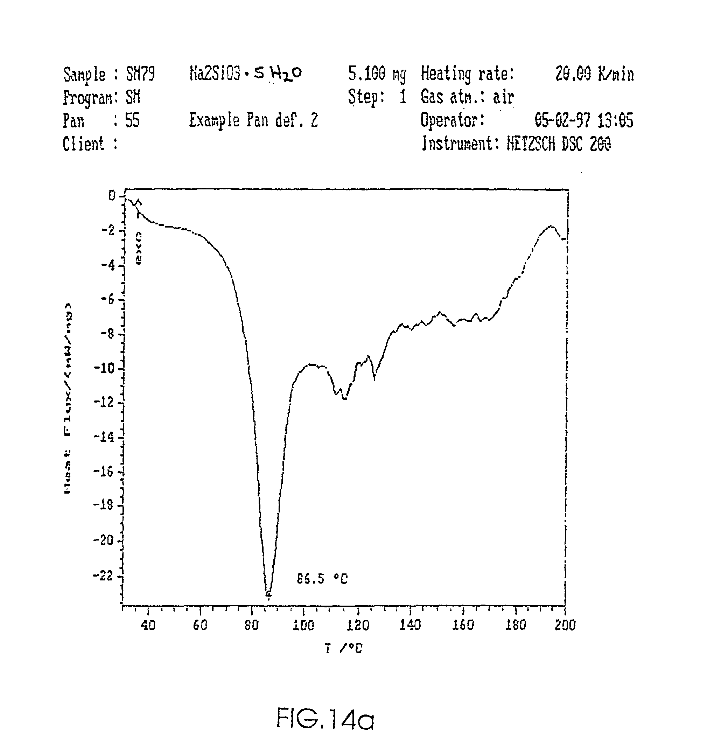

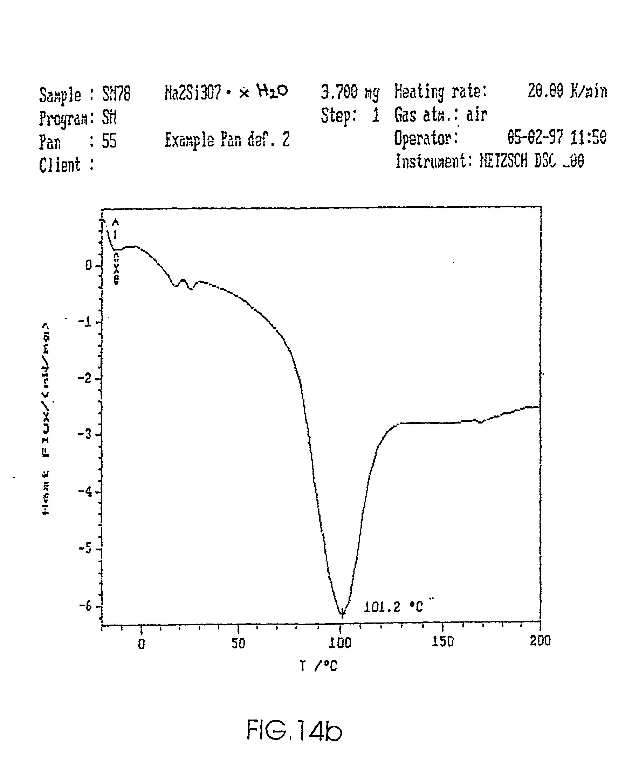

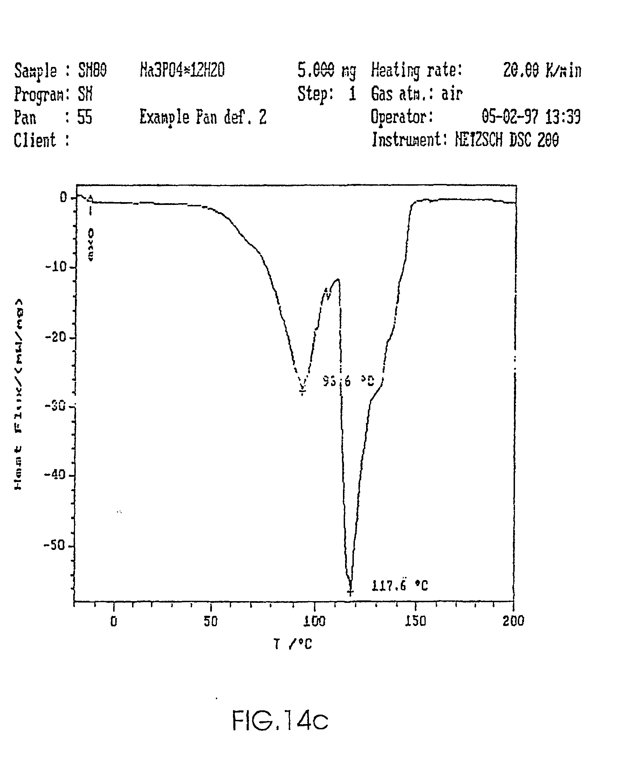

Na₂SiO₃•5 H₂O entwässert im wesentlichen bei 86,5 °C (FIG. 14 a). Bei Na₂Si₃O₇•X H₂O geschieht dies erst bei 101,2 °C (FIG. 14 b).

Bei Na₃PO₄•12 H₂O wird eine mehrstufige Entwässerung beobachtet, die hauptsächlich bei 93,6 °C und 117,6 °C erfolgt (FIG. 14 c).

### 2. Transdermale therapeutische Systeme (TTS)

Alle im Rahmen der Beispielsysteme verwendeten aktivierenden Mittel und therapeutischen Mittel wurden vor ihrer Verwendung in einer Schlagkreuzmühle gemahlen und durch ein Sieb mit 50 µm Maschenweite gesiebt. Dies war erforderlich, um die Zubereitungen in dünnen Schichten ausstreichen zu können und eine einheitliche Verteilung im Endprodukt zu erzielen.

### 2.1. Beispiel-TTS mit dem Wirkstoff SDZ ENA713

Ein Beispiel-TTS wurde zur Abgabe des Wirkstoffs SDZ ENA713 an die Haut entwickelt. Es handelt sich um eine Forschungssubstanz des Unternehmens Novartis zur Behandlung der Alzheimerschen Erkrankung.

Die basische Substanz wurde in Form des Hydrogentartrat-Salzes eingesetzt. In einem dem System A entsprechenden Aufbau wurde dieses Wirkstoffsalz gemeinsam mit dem Aktivator Natriummetasilikat in einen Haftkleber auf Silikonbasis eingearbeitet.

Zusammensetzung der haftklebenden Schicht (Gew.-% der getrockneten Matrix):

| Formulierung | I | II | III |
|---|---|---|---|
| ENA 713 Hydrogentartrat | 10 | 10 | 10 |
| Na₂SiO₃ | --- | 3 | --- |
| Na₂SiO₃•5 H₂O | --- | --- | 3 |
| Bio-PSA Q7-4301 | 90 | 87 | 87 |

Bio-PSA Q7-4301 ist ein medizinischer Haftkleber auf Basis von Polydimethylsiloxan (Dow Corning).

Der Wirkstoff und gegebenenfalls der Aktivator wurden der Lösung des Klebers in Benzin zugegeben. Unter Rühren wurde eine homogene Dispersion hergestellt.

Diese Dispersion wurde auf einen geeigneten flächigen Träger beschichtet (im Beispiel ScotchPak 1022, ein Polyethylenterephthalatfilm der Fa. 3M mit einer dehäsiven Beschichtung auf Basis fluorierter Polymere).

Die Trocknung erfolgte 10 Minuten bei Raumtemperatur und 10 Minuten bei 50 °C in einem Ablufttrockenschrank. Das Flächengewicht der Matrix betrug typischerweise 60 g/m².

Die offenliegende Seite der getrockneten Matrix wurde mit einer geeigneten Folie laminiert (im Beispiel Hostaphan RN 15, ein Polyethylenterephthalatfilm der Fa. Hoechst).

Mit den Formulierungen I-III wurden In-vitro-Permeationsversuche am Modell der Kuheuterhaut durchgeführt (n=3). Es handelte sich um statische, zweikammerige Diffusionszellen vom Typ der modifizierten Franz-Zelle, wie sie auf dem Gebiet der TTS-Forschung und - Entwicklung allgemein bekannt ist.

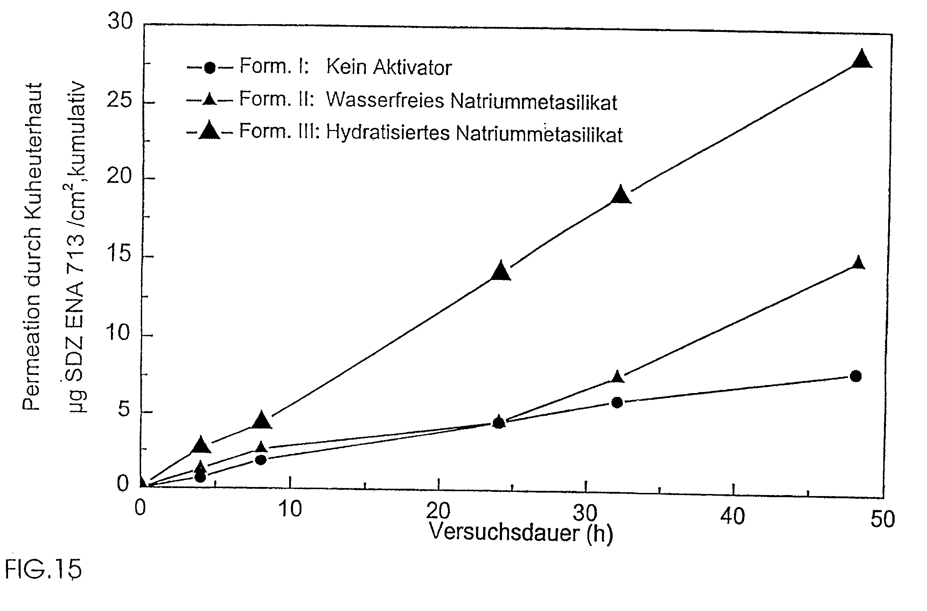

Die erhaltenen Permeationsprofile zeigt FIG. 15.

Während das Wirkstoffsalz ohne Aktivatorzusatz nur in sehr geringer Menge freigesetzt wird, kommt es unter Einfluß der Aktivatoren zu einer gesteigerten Permeation.

Der Aktivator erzielt dabei im hydratisierten Zustand (Pentahydrat) eine deutlich früher einsetzende und insgesamt höhere Freisetzung des Wirkstoffes gegenüber dem wasserfreien Aktivator.

### 2.2. Beispiel-TTS mit dem Wirkstoff Ropinirol

Ropinirol ist ein basischer Wirkstoff der Fa. SmithKline Beecham zur Behandlung der Parkinsonschen Krankheit. Ropinirol ist in Form der freien Base chemisch sehr instabil und eignet sich daher praktisch kaum für die Verarbeitung und Lagerung in einem TTS.

Es wurde ein feuchtigkeitsaktivierbares TTS entwickelt, das anstelle der freien Base das Hydrochlorid enthält. Als Aktivatoren wurden Natriumtrisilikat und verschiedene basische Natriumphosphate erprobt.

Zusammensetzung der haftklebenden Schicht
(Gew.-% der getrockneten Matrix):

| Formulierung | IV | V | VI | VII | VIII | IX |
|---|---|---|---|---|---|---|
| Ropinirol HCl | 10,0 | 10,0 | 10,0 | 10,0 | 10,0 | 10,0 |
| Na₂Si₃O₇ | --- | --- | --- | --- | --- | 6,0 |
| Na₂Si₃O₇•X H₂O* | 6,0 | --- | --- | ---- | --- | --- |
| Na₂SiO₃•5 H₂O | --- | 5,2 | --- | --- | --- | --- |
| Na₂HPO₄•2 H₂O | --- | --- | 6,0 | 3,0 | --- | --- |
| Na₃PO₄•12 H₂O | --- | --- | --- | 6,4 | 6,4 | --- |
| Isopropylmyristat | 1,0 | 1,0 | 1,0 | 1,0 | 1,0 | 1,0 |
| Bio-PSA Q74301 | 83,0 | 83,3 | 83,0 | 79,6 | 82,6 | 83,0 |

| | | | | | | |
|---|---|---|---|---|---|---|
| *Das gebundene Wasser entspricht 10 Gew.-% der Substanz. | | | | | | |

Der Wirkstoff und gegebenenfalls der Aktivator wurden der Lösung des Klebers in Benzin zugegeben, in welche zuvor das flüssige Isopropylmyristat eingerührt wurde. Unter Rühren wurde eine homogene Suspension hergestellt.

Diese Suspension wurde auf einen geeigneten flächigen Träger beschichtet (im Beispiel ScotchPak 1022).

Die Trocknung erfolgte 10 Minuten bei Raumtemperatur und 10 Minuten bei 80 °C in einem Ablufttrockenschrank. Das Flächengewicht der getrockneten Matrix betrug typischerweise 60 g/m².

Die offenliegende Seite der getrockneten Matrix wird mit einer geeigneten Folie laminiert (im Beispiel Hostaphan RN 15).

Mit den Formulierungen IV-VIII wurden In-vitro-Permeationsversuche an menschlicher Vollhaut in modifizierten Franz-Zellen durchgeführt (n=3).

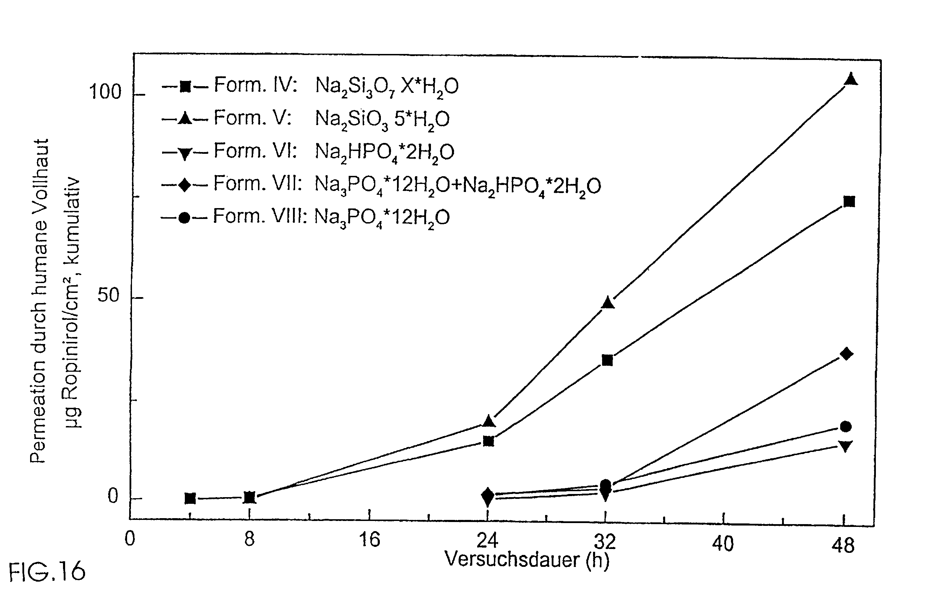

Die erhaltenen Permeationsprofile zeigt FIG. 16.

Die verwendeten Silikate erweisen sich grundsätzlich als Aktivatoren den verwendeten Phosphaten deutlich überlegen. Unter den phosphathaltigen Rezepturen zeigt die Mischung der beiden basischen Phosphate (VII) überraschend die besten Ergebnisse, obwohl das reine Triphosphat (VIII) unter Feuchtigkeitsaufnahme stärker basisch reagieren und damit eine verstärkte Aktivierung von Ropinirol bewirken sollte.

Die weitere Untersuchung ergab, daß sich der Hydratwassergehalt des Natriumtrisilikates eindeutig positiv auf die Aktivatorfunktion auswirkt.

Wird dieser Hilfsstoff vor seiner Anwendung 2 Stunden lang bei 130 °C entwässert (Formulierung IX), so verzögert sich der Aktivierungsprozeß.

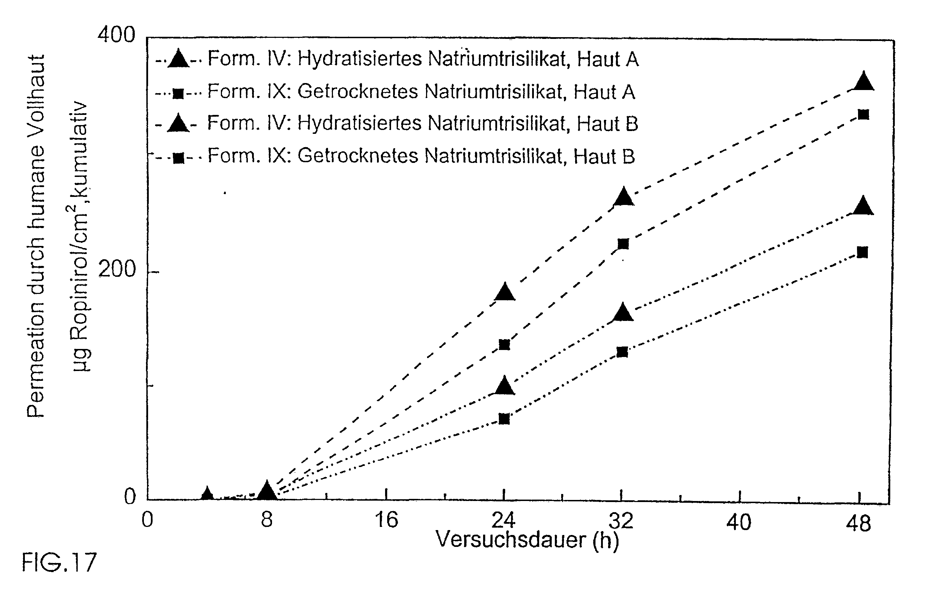

Dieser Befund konnte in vitro an humaner Vollhaut von 2 verschiedenen Individuen bestätigt werden (n=3, Fig. 17). Allein der Hydratwassergehalt bewirkte eine Erhöhung der Permeation nach 24 Stunden um 33 % bzw. 37 % gegenüber der wasserfreien Form des Aktivators.

Für herkömmliche und für feuchtigkeitsaktivierbare TTS mit dem Wirkstoff Ropinirol wurde die Stabilität untersucht. Folgende Formulierungen wurden getestet:

| Formulierung | X | XI | XII | XIII |
|---|---|---|---|---|
| Ropinirol Base | 8,0** | --- | --- | --- |
| Ropinirol HCl | --- | 6,3** | 10,0 | 10,0 |
| Na₂Si₃O₇•X H₂O* | --- | 2,9 | 6,0 | --- |
| Na₂HPO₄•2 H₂O | --- | --- | --- | 3,0 |
| Na₃PO₄•12 H₂O | --- | --- | --- | 6,4 |
| Isopropylmyristat | 2,0 | 2,0 | 2,0 | 2,0 |
| Bio-PSA Q7-4301 | 90,0 | 88,8 | 82,0 | 88,6 |

| | | | | |
|---|---|---|---|---|
| * Das gebundene Wasser entspricht 10 Gew.-% der Substanz. | | | | |
| ** Die freie Base gelöst enthaltende Rezepturen richten sich im Wirkstoffgehalt nach der im jeweiligen Prozeß maximal einzubringenden Menge. Bei kristallin suspendiertem Wirkstoffsalz wurde der Gehalt frei gewählt auf 10 % festgesetzt. | | | | |

Formulierung X wurde durch Emulgierung von öliger Ropinirol-Base in der Benzin-Kleberlösung und Beschichtung sowie Trocknung dieser Lösung hergestellt.

Ropinirol-Base wurde zunächst in folgender Weise aus dem Hydrochlorid-Salz isoliert:

Eine Lösung von 10,0 g Ropinirol-hydrochlorid in 100 ml Wasser wurde durch Zutropfen einer 5 N wäßrigen MaOH-Lösung auf einen pH-Wert von 10-11 eingestellt.

Dieser Ansatz wurde zweimal hintereinander mit je 50 ml Diethylether extrahiert, die beiden Etherphasen wurden vereinigt.

Die etherische Lösung von Ropinirol-Base wurde mit wasserfreiem Na₂SO₄ getrocknet, abfiltriert und anschließend im Stickstoffstrom zur Trockene eingeengt.

Der ölige Rückstand wurde im Exsikkator nachgetrocknet und schließlich bei 4 °C im Kühlschrank kristallisieren gelassen. Die Ausbeute betrug 8,94 g Ropinirol-Base oder 99 % der Theorie. Der Schmelzpunkt wurde zu 73 °C bestimmt (DSC), die Reinheit betrug 98 % (HPLC).

Bei Formulierung XI wurden Ropinirol HCl und Natriumtrisilikat zunächst mit wenig Ethanol vermischt und 18 Stunden unter Rühren bei Raumtemperatur unter Lichtabschluß reagieren gelassen. Diese Vorlösung wurde dann in entsprechender Menge mit der Benzin-Lösung des Klebers vermischt, beschichtet und getrocknet.

Die Herstellung der Formulierungen XII und XIII folgt den Ausführungen zu den Formulierungen IV bis VIII.

Für die Bedingungen der Beschichtung und Trocknung sowie die verwendeten Folienmaterialien bei den Formulierungen X bis XIII siehe ebenfalls die Formulierungen IV bis VIII.

Bereits nach einem Monat Lagerdauer ergibt sich folgendes Bild (n=6):

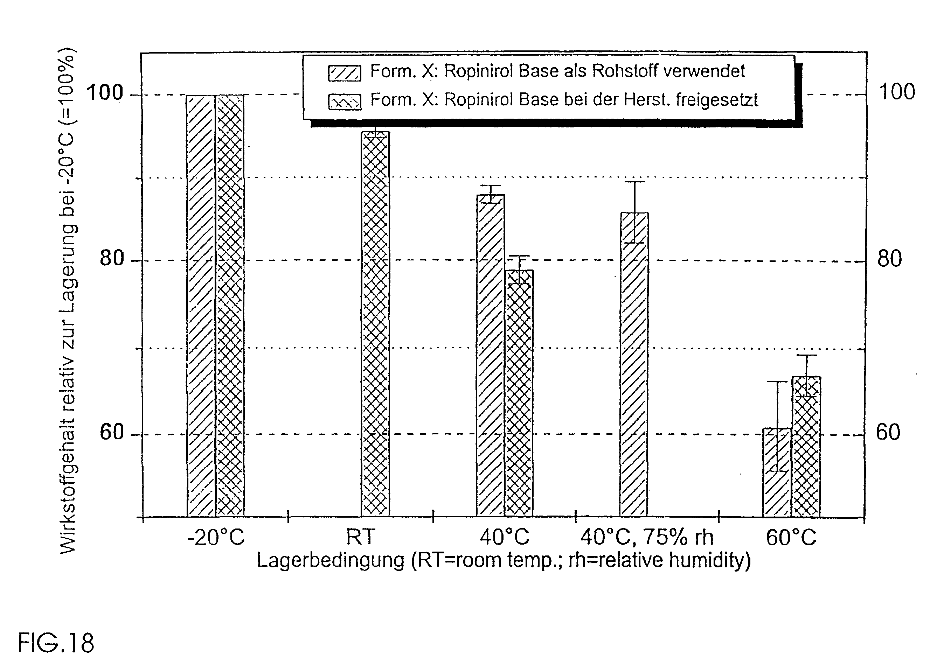

Die freie Base Ropinirol erweist sich selbst bei Verwendung eines reinen Silikonklebers ohne weitere Zusatzstoffe als zu instabil für ein vermarktbares Produkt (Formulierung X, FIG. 18).

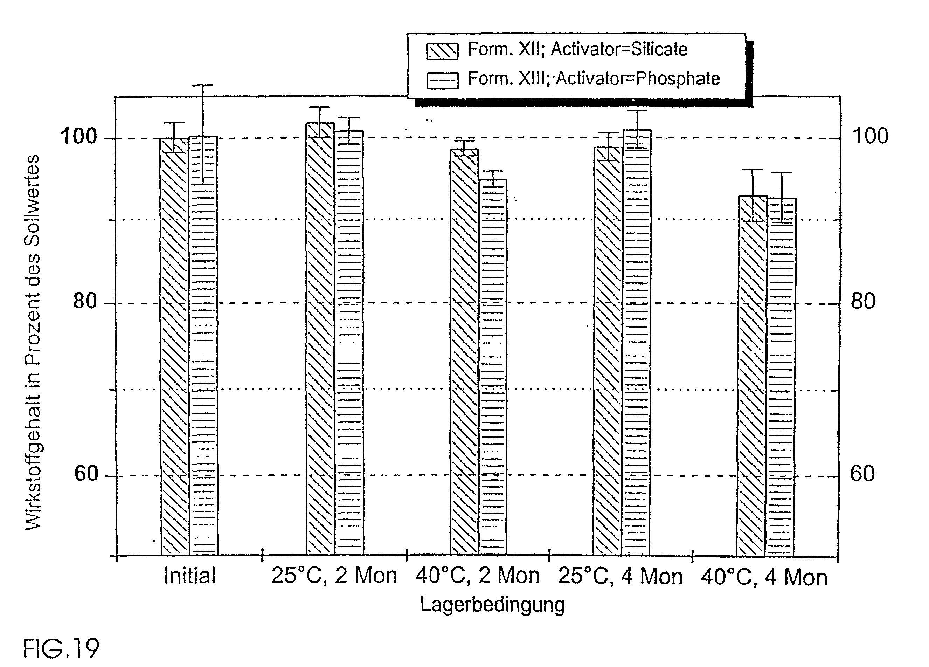

Dies gilt auch, wenn Ropinirol zwar in der Salzform des Hydrochlorides eingesetzt, jedoch daraus schon während der Herstellung des TTS durch den Aktivator die Base freigesetzt wird (Formulierung XI, FIG. 19).

Bei Verwendung von wäßrigem Natriumtrisilikat genügt bereits die Anwesenheit von Ethanol (welches in gewöhnlicher Qualität immer geringe Mengen Wasser enthält), um die Umsetzung von Ropinirol-hydrochlorid zur Base ablaufen zu lassen.

Diese leicht ablaufende Reaktion führt zu der Erkenntnis, daß zur Herstellung der erfindungsgemäßen TTS möglichst unpolare, aprotische Lösungsmittel eingesetzt werden sollten. So kann ein unerwünscht vorzeitiger Ablauf der Umsetzung des Wirkstoffsalzes zur Base verhindert werden.

Solche Lösungsmittel sind vorzugsweise Ethylacetat, Pentan, Hexan, Cyclohexan, Heptan, Octan, Toluol und Xylol, Dichlormethan und Chloroform sowie Benzin verschiedener Siedebereiche.

Die in Benzin-Lösung angesetzten feuchtigkeitsaktivierbaren Rezepturen XII und XIII zeigen anhand der Daten nach 2 und 4 Monaten Lagerung eine erheblich bessere und für ein vermarktbares Produkt genügende Stabilität (n=6, Fig. 19). Die Freisetzung von Base erfolgt hier offenbar, wenn überhaupt, nur zu einem vernachlässigbaren Anteil bei der Herstellung des TTS.

Es wurde bei Formulierungen mit dem Wirkstoff Ropinirol weiterhin gefunden, daß der Verlauf der Feuchtigkeitsaktivierung offenbar durch geringe Mengen von bestimmten Zusatzstoffen modifiziert werden kann. So bewirkt bereits der Zusatz sehr geringer Mengen von Isopropylmyristat eine beschleunigte Aktivierung und dementsprechend erhöhte Gesamtpermeation in vitro. Folgende Rezepturen wurden auf Kuheuterhaut in vitro getestet (n=3):

| Formulierung | XIV | XV | XVI | XVII | XVIII |
|---|---|---|---|---|---|
| Ropinirol HCl | 10,0 | 10,0 | 10,0 | 10,0 | 10,0 |
| Na₂Si₃O₇•X H₂O* | 6,0 | 6,0 | 6,0 | 6,0 | 6,0 |
| Isopropylmyristat | --- | 0,5 | 1,0 | 2,0 | 4,0 |
| Bio-PSA Q7-4301 | 84,0 | 83,5 | 83,0 | 82,0 | 80,0 |

| | | | | | |
|---|---|---|---|---|---|
| *Das gebundene Wasser entspricht 10 Gew.-% der Substanz. | | | | | |

Die Herstellung erfolgte gemäß den Ausführungen zu den Formulierungen IV bis VIII.

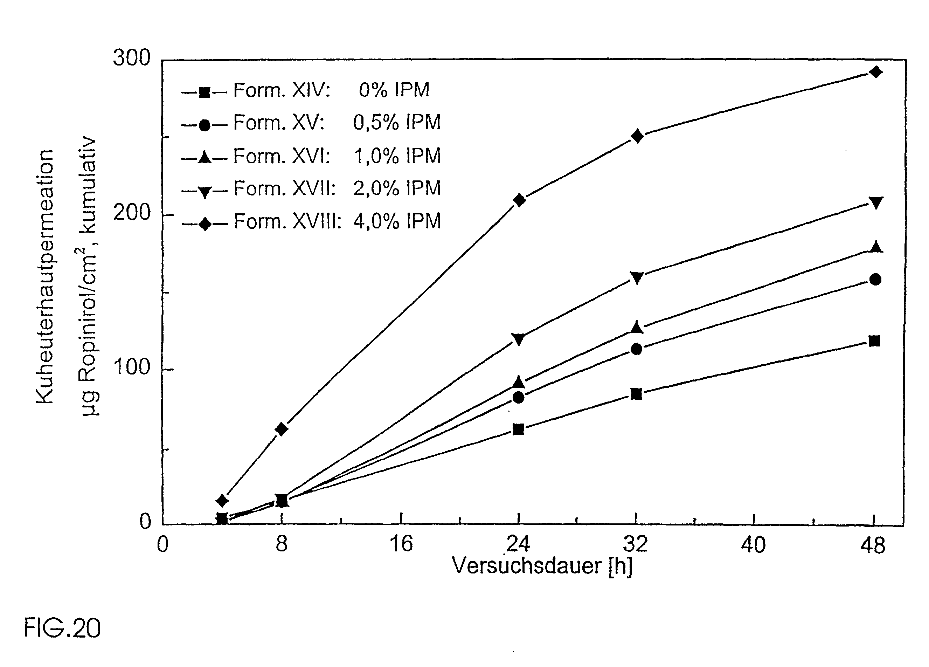

Im Resultat führen schon überraschend geringe Zusätze von Isopropylmyristat zu einer beschleunigten Aktivierung. Der Effekt ist deutlich mit der Menge von Isopropylmyristat korreliert (FIG. 20).

Zwar wurde Isopropylmyristat in der Vergangenheit in vielen Fällen als Permeationsbeschleuniger beobachtet und beschrieben. Jedoch wurde ein solcher, den Hautzustand beeinflussender Effekt, bisher nie für Konzentrationen von nur 0,5 % gefunden. Wahrscheinlicher ist ein die unter Zutritt von Feuchtigkeit ablaufende Säure-Base-Reaktion modulierender Effekt, der hier genutzt werden kann. Daraus ergibt sich die Möglichkeit, die erfindungsgemäßen, feuchtigkeitsaktivierbaren Systeme durch geeignete Zusatzstoffe im Verlauf der Aktivierung zu modulieren.

## Patentansprüche

1. Therapeutisches System zur seitlichen und steuerbaren Abgabe von wenigstens einem therapeutischen Wirkstoff an einen menschlichen oder tierischen Organismus mittels Diffusion durch die Haut oder Schleimhaut, wobei der Wirkstoff für die Herstellung und Lagerung zunächst in einer ersten Zustandsform vorliegt, in der er chemisch stabil und für die Haut oder Schleimhaut nicht ausreichend permeabel ist, während er am Applikationsort durch Zutritt von Feuchtigkeit in eine zweite Zustandsform umgewandelt wird, in der er für die Diffusion durch die Haut oder Schleimhaut geeignet ist und in den Organismus aufgenommen wird, **dadurch gekennzeichnet, daß** er in der ersten Zustandsform im System als pharmazeutisch akseptables Salz enthalten ist, das als ungelöster Feststoff dispergiert vorliegt, und das bei Zutritt von Feuchtigkeit und durch ebenfalls im System enthaltenes aktivierendes Mittel chemisch in die zweite Zustandsform einer Säure oder Base umgewandelt wird, die gegenüber der Salzform beschleunigt und vermehrt von der Haut oder Schleimhaut in den Organismus aufgenommen wird, wobei das aktivierende Mittel eine in wäßriger Lösung sauer oder basisch reagierende Festsubstans oder eine Mischung mehrerer solcher Substanzen ist, walches, bezogen auf seine Gewichtsmenge, einen Wasseranteil von mindestens 5 % in seine Festkörperstruktur eingelagert oder daran gebunden enthält, und wobei Wirkstoffsals und aktivierendes Mittel getrennt voneinander vorliegen.

2. Therapeutisches System nach Anspruch 1, **dadurch gekennzeichnet, daß** das aktivierende Mittel in einer hydratisierten, jedoch ungelösten chemischen Zustandsform eingesetzt wird, wobei es sich bevorzugt um Hydrate des aktivierenden Mittels handelt, die als ungelöster Feststoff im System vorliegen.

3. Therapeutisches System nach Anspruch 1 oder 2, **dadurch gekennzeichnet, daß** es sich bei der Feuchtigkeit am Applikationsort um von der Haut aktiv über Schweißdrüsen abgegebene Flüssigkeit oder um über die Hautoberfläche passiv gasförmig abgegebenen Wasserdampf und im Falle von Schleimhaut um von Drüsen sezernierte Körperflüssigkeiten handelt.

4. Therapeutisches System nach Anspruch 1 oder 2, **dadurch gekennzeichnet, daß** der therapeutische Wirkstoff in einer Menge von 0,5 bis 50 %, vorzugsweise 1,0 bis 20 % des Gesamtgewichtes enthalten ist.

5. Therapeutisches System nach einem der vorangehenden Ansprüche, **dadurch gekennzeichnet, daß** das aktivierende Mittel, bezogen auf die Stöchiometrie der chemischen Reaktion, mit dem therapeutischen Wirkstoff in einem Verhältnis von 0,1 bis 10,0, vorzugsweise 0,2 bis 2,0 zum therapeutischen Wirkstoff enthalten ist.

6. Therapeutisches System nach einem der vorangehenden Ansprüche, **dadurch gekennzeichnet, daß** sowohl der therapeutische Wirkstoff als auch das aktivierende Mittel im wesentlichen in ungelöster Form enthalten sind.

7. Therapeutisches System nach Anspruch 6, **dadurch gekennzeichnet, daß** in ungelöster Form vorliegende Mittel Partikelgrößen zwischen 1 und 200 µm, vorzugsweise zwischen 2 und 50 µm aufweisen.

8. Therapeutisches System nach Anspruch 7, **dadurch gekennzeichnet, daß** es unter Zutritt von Feuchtigkeit im wesentlichen zu einer Auflösung des salzförmigen therapeutischen Mittels kommt, welches dann im System zu dem aktivierenden Mittel diffundiert und dort der Umwandlung in die Form der mit dem Salz chemisch korrespondierenden Säure oder Base unterliegt.

9. Therapeutisches System nach Anspruch 7, **dadurch gekennzeichnet, daß** es unter Zutritt von Feuchtigkeit im wesentlichen zu einer Auflösung des aktivierenden Mittels kommt, welches dann im System zu dem salzförmigen therapeutischen Mittel diffundiert und dieses in die Form der chemisch korrespondierenden Säure oder Base umwandelt.

10. Therapeutisches System nach Anspruch 7, **dadurch gekennzeichnet, daß** sich der therapeutische Wirkstoff und das aktivierende Mittel in einer gemeinsamen, schichtförmigen Matrix gleichmäßig dispergiert befinden.

11. Therapeutisches System nach Anspruch 10, **dadurch gekennzeichnet, daß** eine schichtförmige Matrix oder eine Steuerschicht haftklebende oder mucoadhäsive Eigenschaften besitzt.

12. Therapeutisches System nach Anspruch 7, **dadurch gekennzeichnet, daß** der therapeutische Wirkstoff und das aktivierende Mittel in separaten schichtförmigen Matrices vorhanden sind.

13. Therapeutisches System nach Anspruch 12, **dadurch gekennzeichnet, daß** eine der beiden Matrices unmittelbar mit der Haut oder Schleimhaut in Kontakt ist.

14. Therapeutisches System nach Anspruch 13, **dadurch gekennzeichnet, daß** die zum Kontakt mit der Haut vorgesehene Matrix haftklebende oder mucoadhäsive Eigenschaften besitzt.

15. Therapeutisches System nach Anspruch 1, **dadurch gekennzeichnet, daß** es eine Steuerschicht aufweist, die von therapeutischem Wirkstoff und aktivierendem Mittel frei ist.

16. Therapeutisches System nach Anspruch 15, **dadurch gekennzeichnet, daß** die Steuerschicht so angeordnet ist, daß sie sich bei der Applikation des Systems in unmittelbarem Kontakt mit der Haut oder Schleimhaut befindet.

17. Therapeutisches System nach Anspruch 15, **dadurch gekennzeichnet, daß** die Steuerschicht derart ausgebildet und angeordnet ist, daß sie Ausmaß und Geschwindigkeit der Aufnahme von Feuchtigkeit vom Applikationsort in das System steuert.

18. Therapeutisches System nach Anspruch 15, **dadurch gekennzeichnet, daß** die Steuerschicht derart ausgebildet und angeordnet ist, daß sie Ausmaß und Geschwindigkeit der Diffusion des therapeutischen Mittels in seiner aktivierten Form aus dem System heraus an den Applikationsort steuert.

19. Therapeutisches System nach Anspruch 15, **dadurch gekennzeichnet, daß** die Steuerschicht zwischen dem therapeutischen Wirkstoff und dem aktivierenden Mittel eingelagert ist.

20. Therapeutisches System nach Anspruch 15, **dadurch gekennzeichnet, daß** die Steuerschicht eine Pluralität von in ihrer Zusammensetzung identischen, sphärischen Einzelschichten darstellt, die den ungelöst dispergiert vorliegenden therapeutischen Wirkstoff oder das ungelöst dispergiert vorliegende aktivierende Mittel umhüllen.

21. Therapeutisches System nach Anspruch 15, **dadurch gekennzeichnet, daß** die Steuerschicht so ausgebildet und angeordnet ist, daß sie Ausmaß und Geschwindigkeit der Diffusion des aktivierenden Mittels in seiner gelösten Form zum therapeutischen Wirkstoff hin steuert.

22. Therapeutisches System nach Anspruch 15, **dadurch gekennzeichnet, daß** die Steuerschicht so ausgebildet und angeordnet ist, daß sie Ausmaß und Geschwindigkeit der Diffusion des therapeutischen Wirkstoffs in seiner gelösten Form hin zum aktivierenden Mittel steuert.

23. Therapeutisches System nach einem der vorangehenden Ansprüche, **dadurch gekennzeichnet, daß** es als therapeutischen Wirkstoff Ropinirol-hydrochlorid oder ein anderes, pharmazeutisch akzeptables Salz von Ropinirol enthält.

24. Therapeutisches System nach einem der vorangehenden Ansprüche, **dadurch gekennzeichnet, daß** es als aktivierendes Mittel mindestens eine Substanz aus der Gruppe von Natriumtrisilikat, Natriummetasilikat, Dinatriumphosphat (sekundäres Natriumphosphat) oder Trinatriumphosphat (tertiäres Natriumphosphat)oder des analogen Kaliumsalzes enthält.

25. Therapeutisches System nach einem der vorangehenden Ansprüche, **dadurch gekennzeichnet, daß** es neben therapeutischem Wirkstoff und aktivierendem Mittel noch einen die Aktivierung modulierenden Fettsäureester eines kurzkettigen Alkohols in einer Menge zwischen 0,5 und 5,0 Gewichtsprozent enthält.

26. Therapeutisches System nach Anspruch 25, **dadurch gekennzeichnet, daß** die Ester gebildet sind aus einer gesättigten oder einfach ungesättigten Carbonsäure mit 6 bis 18 Kohlenstoffatomen und einem Alkohol mit 1 bis 3 Kohlenstoffatomen, der maximal eine Hydroxylgruppe pro Kohlenstoffatom aufweist, wobei jedes Alkoholmolekül mit bis zu 3 Fettsäuren verestert sein kann und es sich dabei vorzugsweise um Isopropylmyristat, Isopropylpalmitat oder gesättigte Triglyceride verschiedener Zusammensetzung sowie die Monoglyceride Glycerinmonolaurat und Glycerinmonooleat handelt.

27. Therapeutisches System nach Anspruch 7, **dadurch gekennzeichnet, daß** die schichtförmige Matrix aus einer Formulierung auf Basis von Silikonkautschuk, Polyisobutylen, Polyisopren oder einem Blockcopolymeren von Styrol mit Isopren bzw. Butadien besteht.

## Claims

1. Therapeutic system for timed and controlled release of at least one therapeutic active agent to a human or animal organism by means of diffusion through the skin or mucous membrane, said active agent initially being present, for the purposes of manufacture and storage, in a first state in which it is chemically stable and insufficiently permeable for the skin or mucous membrane, whereas it is converted at the application site into a second state by access of moisture, in which state it is suitable for diffusion through the skin or mucous membrane and in which it is taken up by the organism, **characterized in that** said active agent in said first state is contained in the system as a pharmaceutically acceptable salt which is present dispersed as an undissolved solid and which upon access of moisture and by means of an activating agent, which is likewise contained in the system, is chemically converted into the said second state of an acid or base which is taken up through the skin or mucous membrane into the organism in an accelerated manner and in greater quantities compared to the salt form, said activating agent being a solid substance reacting in aqueous solution as an acid or base, or a mixture of a plurality of such substances, and containing a portion of water of at least 5% in its solid body structure, either intercalated or bound thereto, and active substance salt and activating agent being present separate from each other.

2. Therapeutic system according to Claim 1, **characterized in that** the activating agent is utilized in a hydrated but undissolved chemical state, this preferably being hydrates of the activating agent which are present in the system as an undissolved solid.

3. Therapeutic system according to Claim 1 or 2, **characterized in that** the moisture present at the application site is a liquid which is actively released by the skin via the sweat glands, or water vapour which is passively released in the form of a gas via the skin surface, and that in the case of mucous membranes, said moisture are body liquids secreted by glands.

4. Therapeutic system according to Claim 1 or 2, **characterized in that** the therapeutic active agent is contained in an amount of 0.5 to 50%, preferably 1.0 to 20%, of the overall weight.

5. Therapeutic system according to any one of the preceding claims, **characterized in that** the activating agent, relative to the stoichiometry of the chemical reaction, is contained along with the therapeutic agent at a ratio of 0.1 to 10, preferably 0.2 to 2.0, to the therapeutic agent.

6. Therapeutic system according to any one of the preceding claims, **characterized in that** both the therapeutically active agent and the activating agent are contained in substantially undissolved form.

7. Therapeutic system according to Claim 6, **characterized in that** agents present in undissolved form are of a particle size of between 1 and 200 µm, preferably between 2 and 50 µm.

8. Therapeutic system according to Claim 7, **characterized in that** upon access of moisture there occurs a substantial dissolution of the salt-like therapeutic agent, which therapeutic agent then diffuses towards the activating agent and is subject there to conversion into the form of the acid or base which chemically corresponds to the salt.

9. Therapeutic system according to Claim 7, **characterized in that** under access of moisture there occurs substantial dissolution of the activating agent, which activating agent then diffuses in the system towards the salt-like therapeutic agent and transforms the same into the form of the chemically corresponding acid or base.

10. Therapeutic system according to Claim 7, **characterized in that** the therapeutic agent and the activating agent are present in a joint, layer-like matrix in uniform dispersion.

11. Therapeutic system according to Claim 10, **characterized in that** a layer-like matrix or a control layer possesses pressure-sensitive adhesive or mucoadhesive properties.

12. Therapeutic system according to Claim 7, **characterized in that** the therapeutically active agent and the activating agent are present in separate layer-like matrices.

13. Therapeutic system according to Claim 12, **characterized in that** one of the two matrices is in direct contact with the skin or mucous membrane.

14. Therapeutic system according Claim 13, **characterized in that** the matrix layer intended for contact with the skin possesses pressure-sensitive adhesive or mucoadhesive properties.

15. Therapeutic system according to Claim 1, **characterized in that** it has a control layer which is free of therapeutically active agent and activating agent.

16. Therapeutic system according to Claim 15, **characterized in that** the control layer is arranged such that it is in direct contact with the skin or mucous membrane when the system is applied.

17. Therapeutic system according to Claim 15, **characterized in that** the control layer is configured and arranged such that it controls the extent and speed of the absorption of moisture from the site of application into the system.

18. Therapeutic system according to Claim 15, **characterized in that** the control layer is configured and arranged such that it controls the extent and speed of the diffusion of the therapeutically active agent in its activated form out of the system to the application site.

19. Therapeutic system according to Claim 15, **characterized in that** the control layer is inserted between the therapeutically active agent and the activating agent.

20. Therapeutic system according to Claim 15, **characterized in that** the control layer constitutes a plurality of spherical single layers of identical composition which envelope the undissolved, dispersed therapeutically active agent or the undissolved, dispersed activating agent.

21. Therapeutic system according to Claim 15, **characterized in that** the control layer is configured and arranged such that it controls the extent and speed of the diffusion of the activating agent in its dissolved form towards the therapeutically active agent.

22. Therapeutic system according to Claim 15, **characterized in that** the control layer is configured and arranged such that it controls the extent and speed of the diffusion of the therapeutically active agent in its dissolved form towards the activating agent.

23. Therapeutic system according to any one of the preceding claims, **characterized in that** as therapeutically active agent it contains ropinirole hydrochloride or another, pharmaceutically acceptable, salt of ropinirole.

24. Therapeutic system according to any one of the preceding claims, **characterized in that** as activating agent it contains at least one substance from the group of sodium trisilicate, sodium metasilicate, disodium phosphate (secondary sodium phosphate) or trisodium phosphate (tertiary sodium phosphate) or the analogous potassium salt.

25. Therapeutic system according to any one of the preceding claims, **characterized in that** apart from therapeutically active agent and activating agent it also contains a fatty acid ester of a short-chain alcohol in an amount between 0.5 and 5.0 percent by weight, said fatty acid ester modulating the activation.

26. Therapeutic system according to Claim 25, **characterized in that** the esters are formed from a saturated or mono-unsaturated carboxylic acid with 6 to 18 carbon atoms, and an alcohol with 1 to 3 carbon atoms and having maximally one hydroxyl group per carbon atom, it being possible that each alcohol molecule is esterified with up to 3 fatty acids, and that said esters preferably are isopropyl myristate, isopropyl palmitate or saturated triglycerides of various composition, as well as the monoglycerides glycerin monolaurate and glycerin monooleate.

27. Therapeutic system according to Claim 7, **characterized in that** the layer-like matrix consists of a formulation based on silicone rubber, polyisobutylene, polyisoprene, or a block copolymer of styrene with isoprene or butadiene.

## Revendications

1. Système thérapeutique pour le dégagement réglable et en temps voulu d'au moins une substance thérapeutiquement active à un organisme humain ou animal par diffusion à travers la peau ou à travers une muqueuse, dans lequel la substance active, pour la préparation et l'entreposage, est d'abord présente sous une première forme dans laquelle elle est stable du point de vue chimique et elle n'est pas suffisamment perméable pour la peau ou la muqueuse, tandis qu'elle est transformée, à l'endroit d'application, par apport d'humidité, en une deuxième forme dans laquelle elle est appropriée pour la diffusion à travers la peau ou à travers la muqueuse et dans laquelle elle est absorbée dans l'organisme, **caractérisé en ce que** ladite substance active est contenue, sous la première forme, dans le système, sous la forme d'un sel pharmaceutiquement acceptable qui est présent à l'état dispersé sous la forme d'une substance solide non dissoute et qui est transformé, par apport d'humidité et via un agent d'activation également contenu dans le système, par voie chimique, dans la deuxième forme à savoir celle d'un acide ou d'une base qui, par rapport à la forme de sel, est absorbée plus rapidement et en plus grande quantité par la peau ou la muqueuse dans l'organisme, dans lequel l'agent d'activation représente une substance solide du type à réaction acide ou basique dans une solution aqueuse ou représente un mélange de plusieurs substances de ce type, ledit agent d'activation contenant, rapporté à sa quantité pondérale, une fraction d'eau qui représente au moins 5 % à l'état incorporé dans sa structure de corps solide ou à l'état lié à cette dernière, et dans lequel la substance active sous la forme d'un sel et l'agent d'activation sont présents à l'état dans lequel ils sont séparés l'un de l'autre.

2. Système thérapeutique selon la revendication 1, **caractérisé en ce que** l'agent d'activation est mis en oeuvre sous une forme chimique hydratée quoique non dissoute, dans lequel il s'agit de préférence d'hydrates de l'agent d'activation qui sont présents dans le système sous la forme d'une substance solide non dissoute.

3. Système thérapeutique selon la revendication 1 ou 2, **caractérisé en ce que**, en ce qui concerne l'humidité à l'endroit d'application, il s'agit de liquide qui se dégage de la peau sous une forme active via les glandes sudoripares ou de vapeur d'eau qui se dégage passivement sous une forme gazeuse via la surface de la peau et, dans le cas d'une muqueuse, de fluides corporels sécrétés par des glandes.

4. Système thérapeutique selon la revendication 1 ou 2, **caractérisé en ce que** la substance thérapeutiquement active est contenue en une quantité de 0,5 à 50 %, de préférence de 1,0 à 20 % du poids total.

5. Système thérapeutique selon l'une quelconque des revendications précédentes, **caractérisé en ce que** l'agent d'activation est contenu, rapporté à la stoechiométrie de la réaction chimique, avec la substance thérapeutiquement active dans une proportion de 0,1 à 10,0, de préférence de 0,2 à 2,0 par rapport à la substance thérapeutiquement active.

6. Système thérapeutique selon l'une quelconque des revendications précédentes, **caractérisé en ce que**, aussi bien la substance thérapeutiquement active que l'agent d'activation sont contenus essentiellement sous une forme non dissoute.

7. Système thérapeutique selon la revendication 6, **caractérisé en ce que** les agents présents sous une forme non dissoute, présentent des granulométries entre 1 et 200 µm, de préférence entre 2 et 50 µm.

8. Système thérapeutique selon la revendication 7, **caractérisé en ce que** l'on obtient, par apport d'humidité, essentiellement une dissolution de l'agent thérapeutique sous la forme d'un sel, qui diffuse alors dans le système en direction de l'agent d'activation où il est soumis à la transformation sous la forme d'un acide ou d'une base qui correspond au sel du point de vue chimique.

9. Système thérapeutique selon la revendication 7, **caractérisé en ce que** l'on obtient, par apport d'humidité, essentiellement une dissolution de l'agent d'activation, qui diffuse alors dans le système en direction de l'agent thérapeutique sous la forme d'un sel et transforme ce dernier sous la forme d'un acide ou d'une base correspondant du point de vue chimique.

10. Système thérapeutique selon la revendication 7, **caractérisé en ce que** la substance thérapeutiquement active et l'agent d'activation se trouvent à l'état dispersé de manière uniforme dans une matrice stratifiée commune.

11. Système thérapeutique selon la revendication 10, **caractérisé en ce qu'**une matrice stratifiée ou une couche de réglage possède des propriétés adhésives ou mucoadhésives.

12. Système thérapeutique selon la revendication 7, **caractérisé en ce que** la substance thérapeutiquement active et l'agent d'activation sont présents dans des matrices stratifiées séparées.

13. Système thérapeutique selon la revendication 12, **caractérisé en ce qu'**une des deux matrices entre directement en contact avec la peau ou avec la muqueuse.

14. Système thérapeutique selon la revendication 13, **caractérisé en ce que** la matrice prévue pour entrer en contact avec la peau possède des propriétés adhésives ou mucoadhésives.

15. Système thérapeutique selon la revendication 1, **caractérisé en ce qu'**il présente une couche de réglage qui est exempte de la substance thérapeutiquement active et de l'agent d'activation.

16. Système thérapeutique selon la revendication 15, **caractérisé en ce que** la couche de réglage est disposée de telle sorte que, lors de l'application du système, elle entre directement en contact avec la peau ou avec la muqueuse.

17. Système thérapeutique selon la revendication 15, **caractérisé en ce que** la couche de réglage est réalisée et disposée de telle sorte qu'elle règle la mesure et la vitesse de l'absorption de l'humidité à partir de l'endroit d'application dans le système.

18. Système thérapeutique selon la revendication 15, **caractérisé en ce que** la couche de réglage est réalisée et disposée de telle sorte qu'elle règle la mesure et la vitesse de la diffusion de l'agent thérapeutique sous sa forme activée à partir du système à l'endroit d'application.

19. Système thérapeutique selon la revendication 15, **caractérisé en ce que** la couche de réglage est insérée entre la substance thérapeutiquement active et l'agent d'activation.

20. Système thérapeutique selon la revendication 15, **caractérisé en ce que** la couche de réglage représente plusieurs couches individuelles de forme sphérique et possédant une composition identique qui enveloppent la substance thérapeutiquement active présente à l'état dispersé sous une forme non dissoute ou l'agent d'activation présent à l'état dispersé sous une forme non dissoute.

21. Système thérapeutique selon la revendication 15, **caractérisé en ce que** la couche de réglage est réalisée et disposée de telle sorte qu'elle règle la mesure et la vitesse de la diffusion de l'agent d'activation sous sa forme dissoute en direction de la substance thérapeutiquement active.

22. Système thérapeutique selon la revendication 15, **caractérisé en ce que** la couche de réglage est réalisée et disposée de telle sorte qu'elle règle la mesure et la vitesse de la diffusion de la substance thérapeutiquement active sous sa forme dissoute en direction de l'agent d'activation.

23. Système thérapeutique selon l'une quelconque des revendications précédentes, **caractérisé en ce qu'**il contient, à titre de substance thérapeutiquement active du chlorhydrate de Ropinirol ou un autre sel pharmaceutiquement acceptable de Ropinirol.

24. Système thérapeutique selon l'une quelconque des revendications précédentes, **caractérisé en ce qu'**il contient, à titre d'agent d'activation, au moins une substance choisie parmi le groupe comprenant le trisilicate de sodium, le métasilicate de sodium, le phosphate disodique (phosphate de sodium secondaire) ou le phosphate trisodique (phosphate de sodium tertiaire) ou encore le sel de potassium analogue.

25. Système thérapeutique selon l'une quelconque des revendications précédentes, **caractérisé en ce qu'**il contient, à côté de la substance thérapeutiquement active et de l'agent d'activation, encore un ester d'acide gras d'un alcool à courte chaîne modulant l'activation, en. une quantité entre 0,5 et 5,0 % en poids.

26. Système thérapeutique selon la revendication 25, **caractérisé en ce que** les esters sont formés à partir d'un acide carboxylique saturé ou une fois insaturé contenant de 6 à 18 atomes de carbone et d'un alcool contenant de 1 à 3 atomes de carbone qui présente au maximum un groupe hydroxyle par atome de carbone, dans lequel chaque molécule d'alcool peut être estérifiée avec jusqu'à 3 acides gras et dans lequel, en ce qui concerne ces derniers, il s'agit de préférence du myristate d'isopropyle, du palmitate d'isopropyle ou encore de triglycérides saturés de différentes compositions, ainsi que des monoglycérides que sont le monolaurate de glycérol et le monooléate de glycérol.

27. Système thérapeutique selon la revendication 7, **caractérisé en ce que** la matrice stratifiée est constituée d'une formulation à base de caoutchouc silicone, de polyisobutylène, de polyisoprène ou d'un copolymère séquencé de styrène avec de l'isoprène respectivement du butadiène.
